# EUROPÄISCHE PATENTSCHRIFT

(11) **EP 1 482 934 B1**
(45) Veröffentlichungstag und Bekanntmachung des Hinweises auf die Patenterteilung: **29.03.2006**
(21) Anmeldenummer: 03742914.9
(22) Anmeldetag: 24.02.2003
(51) Int. Cl.: A61K 31/425

(54) **2-METHYLTHIAZOLIDIN-2,4-DICARBONSAEURE UND DEREN SALZE ZUR BEHANDLUNG VON NEURODEGENERATIVEN ERKRANKUNGEN**
2-METHYLTHIAZOLIDINE-2,4-DICARBOXYLIC ACID AND THE SALTS THEREOF USED FOR THE TREATMENT OF NEURODEGENERATIVE DISEASES
ACIDE 2-METHYLTHIAZOLIDIN-2,4-DICARBOXYLIQUE ET SES SELS DESTINES AU TRAITEMENT DE MALADIES NEURODEGENERATIVES

(30) Priorität: 22.02.2002 EP 02003983; 27.02.2002 US 359706 P
(43) Veröffentlichungstag der Anmeldung: 08.12.2004
(73) Patentinhaber: Susilo, Rudy, Dr., 50999 Köln (DE)
(72) Erfinder: SUSILO, Rudy, 50999 Köln (DE)
(74) Vertreter: Arth, Hans-Lothar
(86) Internationale Anmeldenummer: PCT/DE2003/000573
(87) Internationale Veröffentlichungsnummer: WO 2003/072101

(56) Entgegenhaltungen:
- WO-A-98/38994
- DE-A- 2 116 629
- HILL ET AL : "Spontaneous formation of diasterioisomeric 2-methylthiazolidine 2,4,dicarboxylates from cystine esters and related compounds" CHEM COMMUNICATIONS, 1996, Seiten 843-844, XP009012664

## Beschreibung

Die Erfindung betrifft die Verbindungen (2*R*,4*R*)-, (2*S*,4*R*)-2-Methylthiazolidin-2,4-dicarbonsäure sowie (2*RS*,4*R*)-2-Methylthiazolidin-2,4-dicarbonsäure und Salze dieser Verbindungen, und pharmazeutische Zusammensetzungen, welche diese Verbindungen und/oder deren Salze gegebenenfalls neben weiteren Zusatzstoffen enthalten.

Zu den neurodegenerativen Erkrankungen zählen beispielsweise Durchblutungsstörungen des Gehirns, Ischämie, zerebrale Ischämie, Apoplexie, Hirnschlag, Hirnleistungsstörungen, Senile Demenz, Alzheimer, Chorea Huntington und Morbus Parkinson.

Der Morbus Parkinson gehört zu den häufigen neurodegenerativen Erkrankungen des älteren Menschen.

Der Morbus Parkinson (oder idiopathisches Parkinson-Syndrom) ist eine progrediente Erkrankung, die durch die Symptome Ruhetremor, Hypo-/Bradykinese und Rigidität der Muskulatur charakterisiert ist. Neuropathologisch findet sich eine Degeneration der zum Striatum projizierenden dopaminergen Neuronen in der Substantia nigra mit Verminderung des striatalen Dopamingehalts sowie hyaline Einschlußkörperchen (Lewy-Körperchen) in den verbleibenden Neuronen. Ätiologie und Pathophysiologie dieser Erkrankung sind weitgehend unbekannt. Das idiopathische Parkinson-Syndrom zählt zu den häufigen neurologischen Erkrankungen des älteren Menschen. Die ersten Symptome treten gewöhnlich nach dem 50. Lebensjahr auf, junge Menschen sind nur sehr selten betroffen. Die Prävalenz steigt nach dem 60. Lebensjahr exponentiell an, so daß ca. 1 bis 1,5 % der über 60jährigen betroffen sind.

Mittels einer symptomatischen Behandlung durch Substitution des Dopamindefizits ist eine Behandlung der Symptome für einen Zeitraum von 5 bis 10 Jahren möglich. Allerdings kann der Verlauf der Erkrankung, das heißt die Geschwindigkeit der Degeneration der dopaminergen Neuronen, derzeit nicht beeinflußt werden. Die anfangs positiven Berichte über einen neuroprotektiven Effekt des irreversiblen Monoaminoxidase B-Hemmers Selegilin haben sich im Langzeitverlauf nicht bestätigt.

Zur medikamentösen Parkinson-Therapie stehen heute 3 Klassen von Pharmaka zur Verfügung. "

### Dopaminergika

L-DOPA plus Decarboxylase-Hemmstoff (z.B. Madopar®, Nacom®) Dopaminagonisten: Bromocriptin (Pravidel®), Lisurid (Dopergin®), Pergolid (Parkotil®), Dihydrocryptin (Almirid®), Cabergolin, Pramipexol, Ropinirol Monoaminoxidase B-Hemmstoffe: Selegilin (z.B. Deprenyl®)

### Anticholinergika

Benzatropin (Cogentinol®), Trihexyphenidyl (z.B. Artane®), Biperiden (z.B. Akineton®)

### Glutamat-/NMDA-Rezeptor-Antagonisten Amantadine (z.B. PK-Merz®); Memantin.

Als erstes bekanntes Dopaminergikum ist die Vorläufersubstanz des Dopamins, das L-DOPA zu nennen. Sie kann im Gegensatz zu Dopamin die Blut-Hirn-Schranke passieren, wird dann von der dopaminergen Nervenzelle aufgenommen und durch die zytosolische Decarboxylase zu Dopamin umgebaut. Um einen peripheren Umbau zu Dopamin zu verhindem, wird L-DOPA immer mit einem nicht ins Gehim penetrierenden Decarboxylase-Hemmstoff, wie Benserazid (in Madopar®) oder Carbidopa (z.B. Nacom®) kombiniert. In dieser Kombination gilt L-DOPA noch immer als die wirksamste und bestverträglichste dopamimetische Substanz.

Eine weitere Klasse von Dopaminergika sind die direkt auf die striatalen Dopaminrezeptoren wirkenden Dopaminagonisten. Die Wirksamkeit aller Agonisten ist schwächer als die von L-DOPA, es kommt jedoch bei diesen Substanzen, neben den vom L-DOPA bekannten Nebenwirkungen, häufiger zu schwerwiegenden Komplikationen, wie kardiovaskulären Komplikationen sowie Verwirrtheit und Halluzinationen. Aus diesem Grund müssen diese Substanzen bei älteren, multimorbiden Patienten mit Vorsicht eingesetzt werden.

Selegilin (z.B. Movergan®, Deprenyl®) hemmt die Dopamin-abbauende Monoaminoxidase B irreversibel und führt so zu einer Erhöhung des Dopamingehalts im Striatum. Seine Wirkung ist schwach. Es konnte ein neuroprotektiver Effekt im ersten Behandlungsjahr gezeigt werden, der bei Langzeitbehandlung jedoch nicht mehr nachweisbar war.

Anticholinergika reduzieren den Ruhetremor und Rigor, die Hypokinese wird nicht wesentlich beeinflußt. Aufgrund ihres Nebenwirkungsspektrums (Verwirrtheit bis zur Psychose, Glaukom, Harnretention, Schwindel, Müdigkeit), sollten Anticholinergika bei Patienten über 65 Jahren nur bei strenger Indikation, bei Patienten mit kognitiven Defiziten gar nicht verschrieben werden.

Als Glutamat/NMDA-Rezeptorantagonisten stehen die Amantadine (z.B. PK-Merz®) und das nur schwach wirksame Memantin zur Verfügung. Die Wirkungen dieser beiden Substanzgruppen sind im Vergleich mit den oben beschriebenen Dopaminergika schwach.

Wie bereits oben erwähnt, kommt es nach langjähriger Einnahme von Dopaminergika bei vielen Patienten zu motorischen Spätkomplikationen. Zur Behandlung dieser Nebenwirkungen ist eine Stabilisierung der L-DOPA-Blutspiegel (z.B. durch Einsatz von Retard-Präparaten) sinnvoll. Weiterhin ist die Reduktion der L-DOPA-Dosis und höhere Dosierung eines Dopaminagonisten, vorzugsweise mit langer Halbwertszeit (z.B. Pergolid) sinnvoll. Gelegentlich kann eine Monotherapie mit einem Agonisten (auch in Kombination mit Amantadin) versucht werden. In belastenden, langanhaltenden Off-Phasen mit schmerzhaften Dystonien kann bei einigen Patienten nicht auf die subkutane Applikation des Dopaminagonisten Apomorphin verzichtet werden. Insgesamt ist die Behandlung dieser Spätkomplikationen häufig unbefriedigend und erfordert eine intensive Zusammenarbeit von Patient und Arzt. Als letzte Option kann eine stereotaktische Operation erwogen werden.

Eine weitere Komplikation ist das Auftreten von Psychosen unter dopaminerger Medikation. Neben der Tagesdosis des Dopaminergikums wird die Inzidenz und Schwere dieser Komplikation durch das Alter und eventuell andere Läsionen des Gehirns, z.B. eine vaskuläre Enzephalopathie, bestimmt. Diese Medikamenteninduzierten Psychosen äußern sich zunächst in ungewohnt lebhaften Träumen, illusionären Verkennungen und später in optischen, seltener auch akustischen Halluzinationen und Wahnvorstellungen (Dr. med. A. Storch, Dr. med. J. Schwarz, "Geriatrie Praxis", MMV Medizin Verlag GmbH München, Jahrgang 9 (1997) 4, 24-28).

Zudem werden auch operative Parkinsontherapien (Transplantation von Dopaminsynthetisierenden Zellen ins Striatum; intrathekale Applikation von neurotrophen Faktoren; strukturelle oder funktionelle Läsion überaktiver Kerngebiete der Basalganglien) verfolgt, welche jedoch aufgrund teilweise gravierender Nebenwirkungen auch keine Alternative zur medikamentösen Behandlung darstellen.

Der vorliegenden Erfindung liegt die Aufgabe zugrunde physiologisch gut verträgliche Substanzen bereitzustellen, welche zur Prophylaxe und/oder Therapie von neurodegenerativen Erkrankungen eingesetzt werden können und zudem die Nebenwirkungen der bekannten Medikamente vermeiden oder zumindest reduzieren.

Diese Aufgabe wird durch die technische Lehre des unabhängigen Anspruchs 1 sowie der Gegenstände der Ansprüche 5, 6, 9 und 11 der vorliegenden Erfindung gelöst. Weitere vorteilhafte Ausgestaltungen, Aspekte und Details der Erfindung ergeben sich aus den abhängigen Ansprüchen, der Beschreibung und den Beispielen.

Überraschenderweise wurde gefunden, daß (2*R*,4*R*)-2-Methylthiazolidin-2,4-dicarbonsäure, (2*S*,4*R*)-2-Methylthiazolidin-2,4-dicarbonsäure sowie (2*RS*,4*R*)-2-Methylthiazolidin-2,4-dicarbonsäure und auch die Salze dieser Verbindungen erfindungsgemäß zur Prophylaxe und/oder Therapie von neurodegenerativen Erkrankungen verwendet werden können. Als Beispiele für neurodegenerative Erkrankungen seien Parkinson, Chorea Huntington, Durchblutungsstörungen des Gehirns, Ischämie, zerebrale ischärnie, Apoplexie, Hirnschlag, Hirnleistungsstörungen, Senile Demenz oder Alzheimer erwähnt.

Die Salze der oben genannten Verbindung lassen sich durch Zugabe von alkalischen Lösungen herstellen. Als Basen eignen sich beispielsweise Alkali-und Erdalkalihydroxide, Carbonate, Hydrogencarbonate, Phosphate oder Amine.

Besonders geeignet sind Salze von (2*R*,4*R*)-2-Methylthiazolidin-2,4-dicarbonsäure, (2*S*,4*R*)-2-Methylthiazolidin-2,4-dicarbonsäure und/oder (2*RS*,4*R*)-2-Methylthiazolidin-2,4-dicarbonsäure mit Aminosäuren.

Als Aminosäuren können Alanin, Asparagin, Cystein, Glutamin, Phenylalanin, Glycin, Histidin, Isoleucin, Lysin, Leucin, Methionin, Arginin, Serin, Ornithin, Threonin, Valin, Tryptophan, Tyrosin oder Derivate dieser Aminosäuren verwendet werden. Bevorzugt sind basische Aminosäuren wie beispielsweise Lysin, Arginin oder Histidin. Zudem sind basische Salze von Aminosäuren bevorzugt, beispielsweise Natrium-, Kalium- oder Lithiumsalze von Aminosäuren oder Dinatrium-, Dikalium- oder Dilithiumsalze von Glutaminsäure oder Asparaginsäure.

Neben Aminosäuren können Salze auch mit Alkali- und Erdalkalikationen oder Übergangsmetallionen gebildet werden. Verwendet werden können Li⁺, Na⁺, K⁺, Rb⁺, Cs⁺, Be²⁺, Mg²⁺, Ca²⁺, Sc³⁺, Mn²⁺, Fe³⁺, Cu⁺, Cu²⁺, Ag⁺ und Zn²⁺, bevorzugt davon sind Li⁺, Na⁺, K⁺, Cs⁺, Mg²⁺, Ca²⁺ und Zn²⁺ und insbesondere bevorzugt sind Li⁺ und Na⁺.

Insbesondere bevorzugt sind die Lysinsalze von (2*R*,4*R*)-2-Methylthiazolidin-2,4-dicarbonsäure, (2*S*,4*R*)-2-Methylthiazolidin-2,4-dicarbonsäure und/oder (2*RS*,4*R*)-2-Methylthiazolidin-2,4-dicarbonsäure.

Unter (2*RS*,4*R*)-2-Methylthiazolidin-2,4-dicarbonsäure (2-MTDC) soll ein Diastereomerengemisch der (2*S*,4*R*)2-Methylthiazolidin-2,4-dicarbonsäure und (2*R*,4*R*)-2-Methylthiazolidin-2,4-dicarbonsäure verstanden werden.

Die Synthese von nicht diastereomerenreiner 2-Methylthiazolidin-2,4-dicarbonsäure (2-MTDC), ihre Verwendung als Hepatoprotektivum, sowie die Herstellung von nicht diastereomerenreiner 2-Methylthiazolidin-2,4-dicarbonsäure enthaltenden Arzneimitteln in Form von Dragees oder Salben sind aus der DE-A-21 16 629 bekannt. Nicht diastereomerenreines 2-MTDC ist für einige Anwendungen als Arzneimittel vorgeschlagen worden. So offenbart die europäische Patentanmeldung Nr. 989 16 811 die Verwendung von nicht diastereomerenreinem 2-MTDC als Mukolytikum und die europäische Patentanmeldung Nr. 989 16 809 beschreibt ein Kombinationspräparat aus nicht diastereomerenreinem 2-MTDC und Paracetamol.

Bevorzugt zur Prophylaxe und/oder Behandlung von neurodegenerativen Erkrankungen ist die Verwendung von Salzen von 2-MTDC, insbesondere die Salze mit Aminosäuren, wobei die Lysinsalze bevorzugt sind. Die Verwendung der diastereomerenreinen (2*R,*4*R*)-2-Methylthiazolidin-2,4-dicarbonsäure oder (2*S*,4*R)-2*-Methylthiazolidin-2,4-dicarbonsäure in Form ihrer Salze, insbesondere der Salze mit Aminosäuren ist ebenfalls bevorzugt.

Somit können die erfindungsgemäßen Salze von 2-MTDC, (2*R*,4*R*)-2-Methylthiazolidin-2,4-dicarbonsäure und/oder (2*S*,4*R*)-2-Methylthiazolidin-2,4-dicarbonsäure zur Herstellung einer pharmazeutischen Zusammensetzung zur Prophylaxe und/oder Behandlung von neurodegenerativen Erkrankungen, insbesondere von Parkinson, Chorea Huntington, Durchblutungsstörungen des Gehirns, Ischämie, zerebrale Ischämie, Apoplexie, Hirnschlag, Hirnleistungsstörungen, Senile Demenz oder Alzheimer eingesetzt werden. Neben den Salzen eigenen sich auch die salzfreien Verbindungen (2*R*,4*R*)-2-Methylthiazolidin-2,4-dicarbonsäure, (2*S*,4*R*)-2-Methylthiazolidin-2,4-dicarbonsäure sowie (2*RS*,4*R*)-2-Methylthiazolidin-2,4-dicarbonsäure für diese Verwendung.

Zum Nachweis der Wirksamkeit der freien Verbindungen als auch der Salze von 2-MTDC, (2*R*,4*R*)-2-Methylthiazolidin-2,4-dicarbonsäure und/oder (2*S*,4*R*)-2-Methylthiazolidin-2,4-dicarbonsäure wurden Versuche durchgeführt, welche die Auswirkungen von Lysinsalzen auf die Konzentrationen von Dopamin, seine Metaboliten 3,4-Dihydroxyphenylessigsäure (DOPAC) und Homovanillinsäure (HVA) und auf 5-Hydroxytryptamin (5-HT, Serotonin) und seinen Metaboliten 5-Hydroxyindolessigsäure (5-HIAA) in der Substantia nigra und dem Striatum bestimmen sollten.

Außerdem wurde die Konzentration von 3-Methoxytyramin gemessen, einer Indikatorsubstanz für die in vivo Aktivität dopaminerger Neurone. Es wird aus Dopamin durch Methylierung der Hydroxylgruppe in Position 3 durch die Catechol-O-Methyltransferase (COMT) extraneuronal gebildet. Dies bedeutet, daß das Substrat des Enzyms nur Dopamin sein kann, das während der Aktivität der dopaminergen Nervenzellen ausgeschüttet worden ist. Je aktiver diese sind, desto höher ist die Konzentration von 3-Methoxytyramin. Das Lysinsalz von 2-MTDC wurde vier bis fünf Tage vor der Messung von 3-Methoxytyramin intraperitoneal injiziert.

Es konnte nun nachgewiesen werden, daß das Lysinsalz von 2-MTDC zu einer starken Aktivierung der dopaminergen Neurone führt. Diese Wirkung überkompensiert den inaktivierenden Effekt von Malonat. Diese Beobachtung ist deshalb hochinteressant, weil die Wirkung des Lysinsalzes von 2-MTDC lange anhaltend ist.

Das linke und das rechte Striatum wurden als Hirnregionen ausgewählt, um dopaminerge Neurone zu untersuchen, die bekanntermaßen empfindlich auf Neurotoxine reagieren, was beispielsweise zum M. Parkinson führen kann. Die dopaminergen Zellkörper sind in der Substantia nigra lokalisiert und projizieren ihre Axone in das dorsale Striatum. Die weiter unten beschriebene Versuchsdurchführung macht es möglich, degenerative Prozesse im Bereich der Zellfortsätze, die in der Regel zuerst degenerieren und in den Zellkörpern der dopaminergen Neurone, die resistenter gegenüber Noxen sind, getrennt zu untersuchen.

Als neurotoxische Substanz wurde Natriummalonat ausgewählt, da dieses mitochondriale Toxin zu einer akuten Freisetzung von Dopamin im Striatum der Ratte führt. Es wurde nachgewiesen, daß 7 Tage nach der Malonatinjektion die Konzentration von Dopamin im Striatum im Vergleich zu dem kontralateralen Striatum desselben Tieres auf 6% abgesunken war. Der Effekt von Malonat auf die Dopaminkonzentration ist zeit- und dosisabhängig.

Malonat ist ein kompetitiver Hemmstoff der Succinatdehydrogenase (SDH). Dieses mitochondriale Enzym spielt in der neuronalen Energieversorgung eine zentrale Rolle. Es ist am Tricarbonsäurezyklus und an der oxidativen Phosphorylierung beteiligt. Intrastriatale Injektion von Malonat löst eine Läsion aus, die excitotoxisch erscheint und durch Applikation von kompetitiven und nichtkompetitiven Antagonisten am NMDA-Rezeptor verhindert werden kann. Intrastriatale Injektion von höheren Dosen an Malonat führt zu Läsionen von dopaminergen und glutamatergen Zellendigungen und einer erheblichen Abnahme der striatalen ATP-Konzentrationen und der von Dopamin. Die durch Malonat aktivierten excitotoxischen Prozesse, die zum Zelltod führen können, sind an ein bioenergetisches Defizit gekoppelt. Beide Vorgänge sind wahrscheinlich an der Pathogenese zahlreicher neurodegenerativer Erkrankungen, wie beispielsweise Alzheimer, Chorea Huntington und M. Parkinson, beteiligt.

Die erfindungsgemäßen salzfreien Verbindungen als auch die Salze von 2-MTDC, (2R,4R)-2-Methylthiazolidin-2,4-dicarbonsäure und/oder (2*S*,4*R*)-2-Methylthiazolidin-2,4-dicarbonsäure können direkt als salzfreie Verbindung bzw. als Salze oder zubereitet in einer pharmazeutischen Zusammensetzung zur Prophylaxe und/oder Behandlung von neurodegenerativen Erkrankungen, insbesondere von Parkinson, Chorea Huntington, Durchblutungsstörungen des Gehirns, Ischämie, zerebrale Ischämie, Apoplexie, Hirnschlag, Hirnleistungsstörungen, Senile Demenz oder Alzheimer, eingesetzt werden, indem man einem Individuum eine therapeutisch wirksame Menge der jeweiligen salzfreien Verbindung bzw. der jeweiligen Salze verabreicht, welche zur Prophylaxe und/oder Behandlung der jeweiligen neurodegenerativen Erkrankung geeignet ist.

Unter Ischämie wird die Verminderung oder Unterbrechung der Durchblutung eines Organs, Organteils oder Gewebes infolge mangelnder arterieller Blutzufuhr (z.B. durch Thrombose, Embolie, Endarteriitis obliterans, Gefäßspasmus oder Tumoren) verstanden. Zerebrale Ischämie bezeichnet somit die Verminderung oder Unterbrechung der Durchblutung des Gehirns. Die Folgen der Ischämie können Hypoxie, Infarkt sowie Nekrose sein.

Ein weiterer Gegenstand der vorliegenden Erfindung sind pharmazeutische Zusammensetzungen, welche die Verbindungen (2*R*,4*R*)-2-Methylthiazolidin-2,4-dicarbonsäure, (2*S*,4*R*)-2-Methylthiazolidih-2,4-dicarbonsäure, (2*RS*,4*R*)-2-Methylthiazolidin-2,4-dicarbonsäure und/oder Salze dieser Verbindungen als pharmazeutischen Wirkstoff enthalten, gegebenenfalls zusammen mit physiologisch verträglichen Trägern, Hilfsstoffen, Füllstoffen, Geschmacks- oder Farbstoffen und/oder Lösungs- sowie Verdünnungsmitteln.

Die erfindungsgemäß einsetzbaren Verbindungen sowie die erfindungsgemäß einsetzbaren pharmazeutischen Zusammensetzungen eignen sich für eine intravenöse, intraperitoneale, intramuskuläre, subkutane, rektale, transdermale, orale, nasale, buccale, sublinguale oder irgend eine andere Applikation. Besonders bevorzugt ist die orale und parenterale Gabe der erfindungsgemäßen Verbindungen.

Die erfindungsgemäßen Verbindungen werden in einer Dosis von 1 bis 10.000 mg, bevorzugt 10 bis 5.000 mg und insbesondere bevorzugt von 50 bis 1.000 mg verabreicht.

Die erfindungsgemäßen Arzneimittel werden mit den üblichen festen oder flüssigen Trägerstoffen oder Verdünnungsmittels und den üblicherweise verwendeten pharmazeutischen Hilfsstoffen entsprechend der gewünschten Applikationsart mit einer geeigneten Dosierung in bekannter Weise hergestellt. Die bevorzugten pharmazeutischen Formulierungen oder Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Dispersionen, Suspensionen, Depotformen oder Inhalationslösungen.

Selbstverständlich kommen auch parenterale Zubereitungen, wie Injektions- oder Infusionslösungen in Betracht. Weiterhin seien als Zubereitungen auch Suppositorien genannt.

Entsprechende Tabletten können beispielsweise durch Mischen der erfindungsgemäßen Verbindung mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäß einsetzbaren Wirkstoff können zusätzlich geschmacksverbessernde Mittel wie Saccharin, Cyclamat oder Zucker sowie Aromastoffe wie Vanillin oder Orangenextrakt enthalten. Sie können außerdem Suspendierhilfsstoffe wie Natriumcarboxymethylcellulose oder Konservierungsmittel wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. Derivaten davon herstellen.

Verfahren zur Herstellung diverser Formulierungen sowie die verschiedenen Applikationsmethoden sind dem Fachmann bekannt und beispielsweise in "Remington's Pharmaceutical Sciences, Mack Publishing Co., Easton PA" eingehend beschrieben.

### Beispiele

2-MTDC bezeichnet (2*R*,4*R*)-2-Methylthiazolidin-2,4-dicarbonsäure, (2*S*,4*R*)-2-Methylthiazolidin-2,4-dicarbonsäure als auch (2*RS*,4*R*)-2-Methylthiazolidin-2,4-dicarbonsäure, wobei 2-MTDC jedoch bevorzugt für die racemische Verbindung steht.

### Beispiel 1

### Herstellung des Lysinsalzes von 2-MTDC

### 1. Vorbereitung einer 4 N Kaliumacetat-Lösung:

294,45 g (3 Mol) Kaliumacetat (pure, DAB) werden in ca. 400 ml entmineralisiertem Wasser aufgelöst, wobei sich die Lösung erwärmt und nach dem Abkühlen auf 25°C (±5°C) im 1 l-Meßzylinder auf ein Endvolumen von 750 ml eingestellt wird.

### 2. Umsetzung:

In einem 4 l-Rundkolben werden unter Rühren 394,03 g (2,5 Mol) Cys*HCl in 545 ml MeOH suspendiert und mit 1,2 I Isopropanol versetzt. Zunächst werden ca. 125 ml 4 N Kaliumacetat-Lösung zugegeben. Es bilden sich erhebliche Mengen eines weißen Niederschlags. Zu dieser Suspension werden 243 ml (±2,5 ml) Brenztraubensäure gegeben. Der verwendete Meßzylinder wird mit insgesamt 75 ml Isopropanol gespült. Sofort im Anschluß an die Zugabe der Brenztraubensäure wird die restliche Menge 4 N Kaliumacetat-Lösung hinzugegeben, die gut rührbare Suspension erwärmt sich auf ca. 40°C und die Temperatur sinkt langsam im Verlauf von 4 Stunden auf RT. Das Rühren wird beendet und der Ansatz für 16 Stunden (d.h. über Nacht) bei 4°C aufbewahrt. Dabei setzt sich der Niederschlag ab und man erhält einen leicht gelben Überstand.

### 3. Aufarbeitung:

Der Niederschlag wird mittels eines Büchnertrichters abgesaugt, trocken gepreßt und zweimal mit ca. 150 ml entmineralisiertem Wasser aufgerührt, abgesaugt und trocken gepreßt. Anschließend wird im Trockenschrank im Hochvakuum bei ca. 40°C getrocknet.

Das Rohprodukt wird unter intensivem Rühren (kein Magnetrührer!) in ca. 900 ml siedendes entmineralisiertes Wasser eingetragen. Es wird ca. 10 Minuten vorsichtig weiter erhitzt, wobei der zähe Brei intensiv gerührt wird. Anschließend wird die Suspension abgekühlt und nach 3-stündigem Stehen im Eisbad abgesaugt, trocken gepreßt, mit ca. 150 ml eiskaltem entmineralisiertem Wasser aufgerührt, abgesaugt und erneut trocken gepreßt. Anschließend wird im Trockenschrank im Hochvakuum bei ca. 40°C bis zur Gewichtskonstanz getrocknet (Kühlfallenwechsel). Das erhaltene Produkt wird zerkleinert.
Ausbeute: 354g (74% d.Th.)

### 4. Lysinsalz:

10 mmol MTDC und 20 mmol L-Lysin-Base werden in 20 ml Wasser bei Raumtemperatur gelöst. Die klare Lösung wird dann lyophilisiert. Es konnte keine Brenztraubensäure oder freies Cystein in der Lösung nachgewiesen werden.

### Beispiel 2

### Tierversuche:

In einer Versuchsreihe wurde Ratten 2 X 0,8 mmol/kg 2-MTDC Lysinsalz (2-MTDC-Lys) nach 12 h und 48 h intraperitoneal appliziert. 30 Minuten nach der letzten Applikation erhielt die eine Hälfte der Ratten 2 µmol Natriummalonat in das linke Striatum über eine stereotaktisch zuvor implantierte Führungskanüle. Das rechte Striatum diente als intraindividuelles Kontrollgewebe. Vier Tage nach der letzten Applikation, wurden die Konzentrationen der Transmitter und deren Metaboliten bestimmt.

Eine andere Gruppe von Ratten erhielt Lösungsmittel in das linke Striatum und eine dritte Gruppe erhielt 2 µmol Natriummalonat in das linke Striatum.

### Versuchsbeschreibung:

Eine Gruppe von 6 Wistarratten, Charles River, Sulzbach Rosenberg, erhalten innerhalb von 48 h zwei Mal 0,8 mmol/5ml/kg Körpergewicht 2-MTDC-Lys intraperitoneal appliziert. 30 Minuten nach der zweiten Applikation werden 2 µmol Natriummalonat (Sigma) gelöst in physiologischer Kochsalzlösung über eine Nadel mit angeschlossener Präzisionspumpe stereotaktisch bei Vollnarkose (Ketamin 80 mg/kg und Xylasin 6-10 mg/kg i.m.) direkt in das linke Striatum appliziert (Flußrate: 0,5 µl/min, Gesamtvolumen 2 µl).

Vier Tage nach der letzten Applikation wird das Striatum und die Substantia nigra der behandelten Seite sowie der anderen Seite jeweils getrennt excisiert, gewogen, und das Gewebe in 0,1 molarer Perchlorsäure homogenisiert (ein Striatum in 500 µl, eine Substantia nigra in 150 µl) und anschließend bei 13.000 UPM für 5 Minuten (Biofuge 13, Heraeus) zentrifugiert. Der Überstand wird bei 10.000 UMP für 10 Minuten filtriert (Millipore, 0,22 µm Porengröße). Aliquots des Eluenten werden direkt in den HPLC Apparat injiziert. Mittels HPLC-ELCD werden Dopamin, DOPAC, HVA, 5-HT, 5-HIAA und 3-Methoxytyramin aufgetrennt und kolorimetrisch quantifiziert. Da die Konzentration von Dopamin im Striatum relativ zu der Konzentration der Metaboliten hoch ist, wird Dopamin im Striatum getrennt gemessen (Bedingungen: 2 µl Injektionsvolumen, Detektor auf 20 nAmp eingestellt).

Die Bedingungen für die anderen Substanzen und die Substantia nigra sind wie folgt: 5 µl Injektionsvolumen, Detektor: 5 nAmp.

Eine andere Gruppe von Ratten erhält innerhalb von 48 h Lösungsmittel (viermal 50% Propandiol sowie zweimal physiologische Kochsalzlösung jeweils 5 ml/kg) intraperitoneal und 30 Minuten nach der letzten Applikation 2 µmol Natriummalonat intrastriatal links injiziert. Der weitere Versuchsablauf entspricht dem oben beschriebenen.

Eine dritte Gruppe von Ratten erhält innerhalb von 48 h Lösungsmittel (viermal 50% Propandiol und zweimal physiologische Kochsalzlösung jeweils 5 ml/kg) intraperitoneal und 30 Minuten nach der letzten Applikation 2 µl physiologische Kochsalzlösung in das linke Striatum injiziert. Der weitere Versuchsablauf entspricht dem oben beschriebenen.

### Ergebnisse:

Das Lysinsalz von 2-MTDC (2-MTDC-Lys) alleine hat keinen Einfluß auf die Konzentration von Dopamin, DOPAC, HVA. Eine Vorbehandlung mit 2-MTDC-Lys verhindert jedoch die toxische Wirkung von Malonat. 2-MTDC-Lys wirkt neuroprotektiv, da es die Wirkung von Malonat auf die Reduktion der Dopamin- bzw. Dopaminmetabolitenkonzentrationen verhindert.

In der gewählten Dosis und während des Untersuchungszeitraums von vier Tagen gerechnet von der letzten Applikation wirkt 2-MTDC-Lys auf dopaminerge Neurone anscheinend aber nicht auf serotonerge Neurone. Die Wirkungen auf dopaminerge Neurone beinhalten eine lang anhaltende Aktivierung, die möglicherweise auch der Grund für die Neuroprotektion ist. Die Behandlung mit 2-MTDC-Lys verhindert die Neurodegeneration sowohl der Nervenendigungen im Striatum als auch der. Zellkörper in der Substantia nigra (prophylaktisch neuroprotektiv).

Die unter Beispiel 2 beschriebene Versuchsreihe wurde wiederholt, wobei das Lysinsalz von 2-MTDC durch das Natriumsalz ersetzt wurde. Es konnte gezeigt werden, dass auch das Natriumsalz von 2-MTDC vergleichbare Ergebnisse liefert.

### Beispiel 3: therapeutische Wirkung von 2-MTDC und dessen Salzen

In einem anderen tierexperimentellen Modell (Schlaganfall-Modell) konnte weiterhin gezeigt werden (Beispiele 3 und 4), dass 2-MTDC als auch Salze von 2-MTDC eine zerebroprotektive Wirkung haben. Sowohl die freien Säuren von 2-MTDC [(2*R*,4*R*)-2-Methylthiazolidin-2,4-dicarbonsäure, (2*S*,4*R*)-2-Methylthiazolidin-2,4-dicarbonsäure, (2*RS*,4*R*)-2-Methylthiazolidin-2,4-dicarbonsäure] als beispielsweise auch die Lysinsalze von 2-MTDC sind in der Lage, die durch Okklusion der zerebralen Arterie induzierte Infarktgröße in Mäusen (NMRI Mäuse) zu reduzieren. Diese Ergebnisse werden im folgenden am Beispiel des Natriumsalzes von 2-MTDC beschrieben.

### Natriumsalze von 2-MTDC (2-MTDC-Na)

Die folgende Versuchsreihe beschäftigt sich mit der Untersuchung der neuroprotektiven Wirkung von 2-MTDC-Na bei Verabreichung von 2-MTDC-Na nach der Ischämie.

### Versuchsbeschreibung

Pro Testgruppe wurden 13 bis 16 NMRI Mäuse eingesetzt. Die mittlere zerebrale Arterie der Mäuse wurde verschlossen gemäß dem Verfahren von Welsh (F. A. Welsh, T. Sakamoto, A. E. McKee, R. E. Sims, J. Neurochem. 1987, 49, 846 - 851). Dazu wurden die Mäuse mittels Tribromethanol (600 mg / kg Körpergewicht, intraperitoneal) betäubt. Unmittelbar nach der Betäubung wurde ein kleines Loch in den Mausschädel gebohrt, um die mittlere zerebrale Arterie freizulegen. Der Stamm sowie beide Arme der mittleren zerebralen Arterie wurden dauerhaft mittels Elektrokoagulation verschlossen. Währenddessen wurde die Körpertemperatur der Mäuse unter Verwendung einer Wärmelampe auf 37°C ± 1 °C gehalten. Danach wurden die Mäuse weitere 2h bei einer Umgebungstemperatur von 30°C gehalten.

Einer Gruppe von Mäusen wurde 2-MTDC-Na (200 mg / kg 2-MTDC-Na gelöst in 0,9%iger NaCl-Lösung) 15 Minuten und einer anderen Gruppe 60 Minuten nach dem Verschluß der mittleren zerebralen Arterie intraperitoneal injiziert. Eine dritte Gruppe erhielt nur das Vehikel (0,9%ige NaCl-Lösung ohne 2-MTDC-Na).

Für die nachfolgende histologische Untersuchung wurden die Mäuse 2 Tage nach dem Verschluß der mittleren zerebralen Arterie erneut mit Tribromethanol betäubt und erhielten zudem 0,5 ml einer 1,5%igen Lösung von Neutralrot (Toluylenrot, Acros Chimica) intraperitoneal appliziert. Die Gehirne wurden entnommen und in einer Fixierlösung (4% Formalin in Phosphatpuffer bei pH 7,4) für 24h aufbewahrt.

Das Gewebe auf der Oberfläche des Gehirns, welches nicht von Neutralrot eingefärbt worden ist, wurde als vom Infarkt betroffene Oberflächenregion unter Verwendung eines Farbanalysesystems (Kontron, Eching, Deutschland) bestimmt (C. Backhauß, C. Karkoutly, M. Welsch, J. Krieglstein, J. Pharmacol Toxicol Methods, 1992, 27 (1), 27-32).

### Ergebnisse

Infarktbildung konnte nur am kortikalen Gewebe nachgewiesen werden. Zudem korrelierte das Infarktvolumen mit der Infarktoberfläche. 2-MTDC-Na war in der Lage, den vom Infarkt betroffenen Bereich des Gehirns signifikant zu verringern, wobei es wichtig erscheint, 2-MTDC-Na möglichst rasch nach dem Verschluß der mittleren zerebralen Arterie zu applizieren. In der Versuchsreihe, wo 2-MTDC-Na erst nach einer Stunde gerechnet vom Verschluß der mittleren zerebralen Arterie injiziert wurde, zeigte sich nur noch ein stark abgeschwächter Effekt der Infarktverminderung.

Somit konnte gezeigt werden, daß 2-MTDC-Na verabreicht nach der Ischämie weiterhin seine neuroprotektive Wirkung entfaltet, wobei das therapeutische Fenster für die Verabreichung von 2-MTDC-Na ungefähr 60 Minuten gerechnet vom Verschluß der mittleren zerebralen Arterie beträgt.

### Beispiel 4: protektive Wirkung von 2-MTDC und dessen Salzen

Die Versuchsdurchführung gemäß Beispiel 3 wurde wiederholt, wobei 2-MTDC-Na eine Stunde vor dem Verschluss der mittleren zerebralen Arterie (MCA-O: occlusion of the middle cerebral artery) appliziert wurde.

Es konnte gezeigt werden, dass 2-MTDC-Na bei einer Konzentration von 200 mg pro kg Körpergewicht des Versuchstieres das Gehirn der Tiere vor Schäden verursacht durch zerebrale Ischämie schützt, wenn 1 h vor dem Verschluss der mittleren zerebralen Arterie das 2-MTDC-Na intraperitoneal injiziert wird.

Die Versuchsreihen gemäß Beispiel 3 und 4 wurden anstelle von 2-MTDC-Na mit 2-MTDC-Lys als auch mit der freien Säure von 2-MTDC wiederholt. Die Versuche mit 2-MTDC-Lys und der freien Säure von 2-MTDC lieferten ähnliche Ergebnisse wie die Versuche mit 2-MTDC-Na.

## Patentansprüche

1. (2*R*,4*R*)-2-Methylthiazolidin-2,4-dicarbonsäure, (2*S*,4*R*)-2-Methylthiazolidin-2,4-dicarbonsäure und (2*RS*,4*R*)-2-Methylthiazolidin-2,4-dicarbonsäure sowie Salze dieser Verbindungen.

2. Salze gemäß Anspruch 1, **dadurch gekennzeichnet, daß** es sich bei den Salzen um physiologisch verträgliche Substanzen insbesondere mit Aminosäuren handelt.

3. Salze gemäß Anspruch 2, **dadurch gekennzeichnet, daß** es sich um das Lysinsalz handelt.

4. Salze gemäß Anspruch 1 oder 2, **dadurch gekennzeichnet, daß** es sich um Li⁺, Na⁺, K⁺, Cs⁺, Mg²⁺, Ca²⁺ und/oder Zn²⁺ -Salze handelt.

5. Verwendung von (2*R*,4*R*)-2-Methylthiazolidin-2,4-dicarbonsäure, (2*S*,4*R*)-2-Methylthiazolidin-2,4-dicarbonsäure und/oder (2*RS*,4*R*)-2-Methylthiazolidin-2,4-dicarbonsäure sowie deren Salze zur Prophylaxe und/oder Behandlung von neurodegenerativen Erkrankungen.

6. Verwendung von (2*R*,4*R*)-2-Methylthiazolidin-2,4-dicarbonsäure, (2*S*,4*R*)-2-Methylthiazolidin-2,4-dicarbonsäure und/oder (2*R*S,4*R*)-2-Methylthiazolidin-2,4-dicarbonsäure sowie deren Salze zur Herstellung einer pharmazeutischen Zusammensetzung zur Prophylaxe und/oder Behandlung von neurodegenerativen Erkrankungen.

7. Verwendung gemäß Anspruch 5 oder 6, **dadurch gekennzeichnet, daß** es sich bei den neurodegenerativen Erkrankungen um Parkinson, Chorea Huntington, Durchblutungsstörungen des Gehirns, Ischämie, zerebrale Ischämie, Apoplexie, Hirnschlag, Hirnleistungsstörungen, Senile Demenz oder Alzheimer handelt.

8. Verwendung nach einem der Ansprüche 5-7, **dadurch gekennzeichnet, daß** (2*RS*,4*R*)-2-Methylthiazolidin-2,4-dicarbonsäure, (2*S*,4*R*)-2-Methylthiazolidin-2,4-dicarbonsäure, (2*R*,4*R*)-2-Methylthiazolidin-2,4-dicarbonsäure, die Salze von (2*RS*,4*R*)-2-Methylthiazolidin-2,4-dicarbonsäure, von (2*S*,4*R*)-2-Methylthiazolidin-2,4-dicarbonsäure und/oder von (2R,4R)-2-Methylthiazolidin-2,4-dicarbonsäure in einer Dosis von 1 - 10.000 mg, insbesondere von 10 bis 5.000 mg eingesetzt werden.

9. Pharmazeutische Zusammensetzung enthaltend (2*R*,4*R*)-2-Methylthiazolidin-2,4-dicarbonsäure, (2*S*,4*R*)-2-Methylthiazolidin-2,4-dicarbonsäure, (2*RS*,4*R*)-2-Methylthiazolidin-2,4-dicarbonsäure und/oder deren Salze als pharmazeutischen Wirkstoff zusammen mit physiologisch verträglichen Trägern, Hilfsstoffen und/oder Lösungsmitteln.

10. Pharmazeutische Zusammensetzung gemäß Anspruch 9, **dadurch gekennzeichnet, daß** die pharmazeutische Zusammensetzung zur intravenösen, intraperitonealen, intramuskulären, subkutanen, rektalen, transdermalen, oralen, nasalen, buccalen oder sublingualen und insbesondere zur oralen und parenteralen Applikation geeignet ist.

## Claims

1. (2*R*,4*R*)-2-methylthiazolidine-2,4-dicarboxylic acid, (2*S*,4*R*)-2-methylthiazolidine-2,4-dicarboxylic acid and (2*RS*,4*R*)-2-methylthiazolidine-2,4-dicarboxylic acid as well as salts of these compounds.

2. Salts according to claim 1, **characterized in, that** the salts concern physiologically compatible substances especially with amino acids.

3. Salts according to claim 2, **characterized in, that** the lysine salt is concerned.

4. Salts according to claim 1 or 2, **characterized in, that** the Li⁺, Na⁺, K⁺, Cs⁺, Mg²⁺, Ca²⁺ and/or Zn²⁺ salts are concerned.

5. Use of (2R,4R)-2-methylthiazolidine-2,4-dicarboxylic acid, (2*S*,4*R*)-2-methylthiazolidine-2,4-dicarboxylic acid and/or (2*RS*,4*R*)-2-methylthiazolidine-2,4-dicarboxylic acid as well as their salts for prophylaxis and/or treatment of neurodegenerative diseases.

6. Use of (2R,4R)-2-methylthiazolidine-2,4-dicarboxylic acid, (2*S*,4*R*)-2-methylthiazolidine-2,4-dicarboxylic acid and/or (2*RS*,4*R*)-2-methylthiazolidine-2,4-dicarboxylic acid as well as their salts for preparing a pharmaceutic formulation for prophylaxis and/or treatment of neurodegenerative diseases.

7. Use according to claim 5 or 6, **characterized in, that** the neurodegenerative diseases concern Parkinson, Huntington's chorea, cerebral circulatory disorders, ischemia, cerebral ischemia, apoplexy, cerebral apoplexy, brain deficiencies, senile dementia or Alzheimer's disease.

8. Use according to one of the claims 5 - 7, **characterized in, that** (2*R*S,4*R*)-2-methylthiazolidine-2,4-dicarboxylic acid, (2*S*,4*R*)-2-methylthiazolidine-2,4-dicarboxylic acid, (2*R*,4*R*)-2-methylthiazolidine-2,4-dicarboxylic acid, the salts of (2*RS*,4*R*)-2-methylthiazolidine-2,4-dicarboxylic acid, of (2*S*,4*R*)-2-methylthiazolidine-2,4-dicarboxylic acid and/or of (2*R*,4*R*)-2-methylthiazolidine-2,4-dicarboxylic acid are used in a dose rate of 1 - 10,000 mg, especially from 10 to 5,000 mg.

9. Pharmaceutic formulation comprising (2*R*,4*R*)-2-methylthiazolidine-2,4-dicarboxylic acid, (2*S*,4*R*)-2-methylthiazolidine-2,4-dicarboxylic acid, (2*RS*,4*R*)-2-methylthiazolidine-2,4-dicarboxylic acid and/or their salts as pharmaceutically active agent together with physiologically compatible carriers, auxiliary materials and/or solvents.

10. Pharmaceutic formulation according to claim 9, **characterized in, that** the pharmaceutic formulation is suitable for intravenous, intraperitoneal, intramuscular, subcutaneous, rectal, transdermal, oral, nasal, buccal or sublingual and especially for oral and parenteral application.

## Revendications

1. Acide (2R,4R)-2-méthylthiazolidine-2,4-dicarboxylique, acide (2S,4R)-2-méthylthiazolidine-2,4-dicarboxylique et acide (2RS,4R)-2-méthyl-thiazolidine-2,4-dicarboxylique, ainsi que les sels de ces composés.

2. Sels selon la revendication 1, **caractérisés en ce que** ces sels concernent des substances physiologiquement acceptables, en particulier avec des aminoacides.

3. Sels selon la revendication 2, **caractérisés en ce que** le sel de lysine est concerné.

4. Sels selon la revendication 1 ou 2, **caractérisés en ce qu'**il s'agit de sels de Li⁺, Na⁺, K⁺, Cs⁺, Mg²⁺, Ca²⁺ et/ou Zn²⁺.

5. Utilisation d'acide (2R,4R)-2-méthylthiazolidine-2,4-dicarboxylique, d'acide (2S,4R)-2-méthylthiazolidine-2,4-dicarboxylique et/ou d'acide (2RS,4R)-2-méthylthiazolidine-2,4-dicarboxylique ainsi que de leurs sels, pour la prophylaxie et/ou le traitement de maladies neurodégénératives.

6. Utilisation d'acide (2R,4R)-2-méthylthiazolidine-2,4-dicarboxylique, d'acide (2S,4R)-2-méthylthiazolidine-2,4-dicarboxylique et/ou d'acide (2RS,4R)-2-méthylthiazolidine-2,4-dicarboxylique ainsi que de leurs sels, pour la préparation d'une composition pharmaceutique destinée à la prophylaxie et/ou au traitement de maladies neurodégénératives.

7. Utilisation selon la revendication 5 ou 6, **caractérisée en ce que** les maladies neurodégénératives sont la maladie de Parkinson, la chorée de Huntington, des troubles de la circulation du cerveau, l'ischémie, l'ischémie cérébrale, l'apoplexie, l'accident vasculaire cérébral, des troubles de la fonction cérébrale, la démence sénile ou la maladie d'Alzheimer.

8. Utilisation selon l'une quelconque des revendications 5 à 7, **caractérisée en ce qu'**on utilise de l'acide (2R,4R)-2-méthylthiazolidine-2,4-dicarboxylique, de l'acide (2S,4R)-2-méthylthiazolidine-2,4-dicarboxylique, de l'acide (2RS,4R)-2-méthylthiazolidine-2,4-dicarboxylique, des sels d'acide (2R,4R)-2-méthylthiazolidine-2,4-dicarboxylique, d'acide (2S,4R)-2-méthylthiazolidine-2,4-dicarboxylique et/ou d'acide (2RS,4R)-2-méthylthiazolidine-2,4-dicarboxylique à une dose de 1 à 10 000 mg, en particulier de 10 à 5 000 mg.

9. Composition pharmaceutique contenant, à titre de principe actif pharmaceutique, de l'acide (2R,4R)-2-méthylthiazolidine-2,4-dicarboxylique, de l'acide (2S,4R)-2-méthylthiazolidine-2,4-dicarboxylique, de l'acide (2RS,4R)-2-méthylthiazolidine-2,4-dicarboxylique et/ou des sels de ceux-ci, conjointement à des véhicules, adjuvants et/ou solvants physiologiquement acceptables.

10. Composition pharmaceutique selon la revendication 9, **caractérisée en ce que** la composition pharmaceutique est adaptée à une administration intraveineuse, intrapéritonéale, intramusculaire, sous-cutanée, rectale, transdermique, orale, nasale, gingivo-jugale ou sublinguale, et en particulier à une administration orale ou parentérale.
